# EUROPÄISCHE PATENTSCHRIFT

(11) **EP 1 140 634 B1**
(45) Veröffentlichungstag und Bekanntmachung des Hinweises auf die Patenterteilung: **07.05.2003**
(21) Anmeldenummer: 99966941.9
(22) Anmeldetag: 10.12.1999
(51) Int. Cl.: B65B 61/00, B65D 65/42, A61L 9/04

(54) **VERFAHREN ZUR BEDUFTUNG VON VERPACKUNGEN**
METHOD FOR FRAGRANCING PACKAGING
PROCEDE PERMETTANT DE PARFUMER DES EMBALLAGES

(30) Priorität: 19.12.1998 DE 19858858
(43) Veröffentlichungstag der Anmeldung: 10.10.2001
(73) Patentinhaber: Henkel Kommanditgesellschaft auf Aktien, 40589 Düsseldorf (DE)
(72) Erfinder: BARTHEL, Wolfgang, D-40764 Langenfeld (DE); LAHN, Wolfgang, D-47877 Willich (DE)
(86) Internationale Anmeldenummer: EP9909733
(87) Internationale Veröffentlichungsnummer: WO00037319

(56) Entgegenhaltungen:
- EP-A- 0 483 126
- US-A- 4 540 721

## Beschreibung

Die vorliegende Anmeldung beschäftigt sich mit einem Verfahren zur Beduftung von Verpackungen sowie einer Zubereitung, die sich zum Beduften von Verpackungen eignet.

Die Verpackung hat neben dem Schutz des darin enthaltenen Verkaufsartikels oft auch die Funktion, den Artikel in ansprechender Weise zu präsentieren. Hierzu werden Verpackungen üblicherweise so gestaltet, daß sie optisch ansprechend sind und dem Verbraucher bereits wesentliche Informationen über das Produkt liefern. Der Duft eines Produkts ist jedoch eine Information, die Verpackungen kaum vermitteln können, vielmehr verhindern Verpackungen häufig gerade, daß der Kunde den Duft des Produktes wahrnehmen kann. So ist beispielsweise der Duft eines Wasch- und/oder Reinigungsmittels eine wesentliche Information, da dieser Duft auch den Duft des damit gereinigten Materials, insbesondere der Wäsche beeinflußt. Wasch- und/oder Reinigungsmittel werden jedoch häufig in Verpackungen angeboten, die wasser- und damit verbunden meist auch duftundurchlässig sind. Daher besteht das Bedürfnis, die Verpackungen mit dem Duft des jeweiligen Mittels entsprechend zu beduften, damit der Verbraucher bereits beim Öffnen der Verpackung einen Eindruck von der Duftwirkung des Produktes erhält. Insbesondere bei solchen Mitteln, die neben einer duftundurchlässigen Verpackung eine weitere Umverpackung besitzen, kann die beduftete Umverpackung bereits frühzeitig den Dufteindruck vermitteln.

Gegenstand der vorliegenden Erfindung ist dementsprechend ein Verfahren zur Beduftung von Verpackungen, insbesondere Umverpackungen von Produkten, die selbst duftundurchlässig verpackt sind, wobei die Beduftung dem Zweck dient, den Verbraucher einen Eindruck von der Duftwirkung des Produktes zu vermitteln.

Zur Beduftung von Verpackungen schlägt die europäische Patentanmeldung EP-A-004463 das Auftragen oder Aufsprühen von Duftstoffen bzw. Lösungen solcher Duftstoffe auf das Innere von Verpackungskartons vor. Probleme bei Anwendung dieser Methode sind, daß die so aufgebrachten Duftstoffe von den meisten Kartonmaterialien nur langsam aufgenommen werden und dementsprechend am Karton entlanglaufen und sichtbare Flecken bilden. Weiter führt insbesondere das Versprühen der Duftstoffe und der damit verbundenen Duftvernebelung zu erheblichen Substanzverlusten und zu einer Geruchsbelästigung der Umgebung.

Zur Vermeidung solcher Nachteile wird in der deutschen Patentanmeldung DE-A-3201941 vorgeschlagen, den Duftstoff vor dem Auftragen in eine höherviskose Paste einzuarbeiten und diese Paste durch Sprühen, Rakeln oder Streichen auf das Verpackungsmaterial aufzutragen. Bevorzugte Pasten enthalten ein Geliermittel, wobei als Geliermittel hochdisperse Kieselsäure verwendet wird. Auch bei diesem Verfahren ist jedoch die Saugfähigkeit der Verpackungsmaterialien von großer Bedeutung, da auch diese Pasten auf wenig saugfähigen Materialien zu Flecken oder Nasen führen oder als schmierige Schicht zurückbleiben. Bei der Verwendung derartiger saugfähiger Verpackungen hat sich in der Praxis gezeigt, daß durch Wechselwirkungen der Duftstoffe mit den Kartonmaterialien teilweise unangenehme Duftnoten entstehen können.

Ein filmbildendes Mittel zur Beduftung von nicht-saugfähigen Kunststoffmaterialien wird in der Offenlegungsschrift DE-A-3247708 beschrieben. Das Mittel enthält filmbildende, geruchlose, organische Polymere, insbesondere Polyvinylacetat oder Vinylacetat-haltige Copolymere, die Duftstoffe sowie Wasser und/oder weitere Lösungsmittel. Diese Lösungen oder Suspensionen werden durch Aufsprühen, Aufstreichen oder Aufrakeln auf die zu beduftenden Kunststoffmaterialien aufgebracht und bilden nach dem Trocknen einen nicht-klebenden Film, der den Duft langsam abgibt. Zur Viskositätserhöhung und um aus konzentrierten Parfümöl-Emulsionen die Abtrennung der Parfümöle zu verhindern, wird bevorzugt wieder Kieselsäure zugesetzt.

Die Verwendung von Klebstoffen, welche Parfümstoffe enthalten, zur Herstellung von Wasch- und Reinigungsmittelverpackungen aus Pappe und/oder Papierwerkstoffen beschreibt die deutsche Offenlegungsschrift DE-A-3247709. Als Klebstoff wird dabei eine wäßrige Dispersion eines Homo- oder Copolymerisats von Vinylacetat und/oder Vinylpropionat, die eine Viskosität zwischen 800 und 2500 mPas bei Raumtemperatur aufweist, verwendet. Dieser beduftete Klebstoff findet insbesondere zur Verklebung der Kopfklappen von Waschmittelverpackungen Verwendung. Allerdings ist es wesentlich, daß zumindest eine der Flächen, die verklebt werden, zumindest teilweise porös ist, da sonst die Duftstoffe nicht mehr in ausreichendem Maße freigesetzt werden. Die Herstellung der hierzu notwendigen Perforation stellt einen zusätzlichen Verfahrensschritt dar, der eine Umstellung der gesamten Verpackungsproduktion erfordert.

In der älteren Deutschen Patentanmeldung mit dem Aktenzeichen 19810886.9 wird ein Behältnis für wäßrige Bleichmittel auf Hypochloritbasis, bestehend aus einem Kunststoffhohlkörper mit Ausgußöffnung und einem Verschlußdeckel beschrieben, wobei der Verschlußdeckel wenigstens anteilmäßig aus einem Kunststoff besteht, der einen oder mehrere Duftstoffe mit Zitrusaroma verkapselt enthält. Der Deckel gibt die Duftstoffe zeitverzögert in den Innenraum ab, so daß das Bleichmittel auch nach Monaten noch den Zitrusduft aufweist, obwohl der Duft nicht stabil gegen Hypochlorit ist.

Alle diese aus dem Stand der Technik bekannten Verfahren zur Beduftung von Verpackungen setzen bei Raumtemperatur flüssige Zubereitungen der Duftstoffe ein, die nach Aufbringen auf die Verpackung entweder trocknen oder einziehen müssen. Im Interesse der wirtschaftlichen Anwendbarkeit eines Verfahrens zur Verpackungsbeduftung ist es erforderlich, die Beduftung in möglichst kurzer Zeit durchführen zu können. Weiter sollte ein vorteilhaftes Verfahren die oben genannten Nachteile, insbesondere Nachteile in der Verpackungsstabilität und dem Dufteindruck, vermeiden.

Die europäische Patentanmeldung EP-A-0483126 beschreibt hingegen ein Verfahren zur Beduftung von Verpackungen, wobei eine bei Raumtemperatur feste Zubereitung aus Aromaölen und Polymeren bei erhöhter Temperatur in geschmolzenem Zustand auf die Verpackung aufgebracht wird. Ebenso wird eine bei Raumtemperatur feste Zubereitung, die bei erhöhter Temperatur schmilzt und Aromaöle und Polymere enthält, zur Beduftung von Verpackungen offenbart.

Gegenstand der vorliegenden Anmeldung ist dementsprechend ein Verfahren zur Beduftung von Verpackungen gemäss dem Oberbegriff des Anspruchs 1, welches dadurch gekennzeichnet ist, daß Parfüm- und/oder Aromaöle bei erhöhter Temperatur mit einer bei Raumtemperatur festen Dispersion, enthaltend mindestens ein Polymer und mindestens ein Carbonsäuresalz, in Wasser gemischt werden und diese bei Raumtemperatur ebenfalls feste Mischung bei einer Temperatur von mindestens 50 °C und maximal 85 °C auf die Verpackung aufgebracht wird, wobei die Mischung bei der Verarbeitungstemperatur und einer Scherrate von 100 s⁻¹ eine Viskosität von maximal 10000 mPas besitzt.

Gegenstand der vorliegenden Anmeldung ist weiterhin eine Zubereitung gemäss Anspruch 6.

Ein Vorteil des erfindungsgemäßen Verfahrens besteht darin, daß hier eine Schmelze verarbeitet wird, die beim Abkühlen direkt nach der Aufbringung innerhalb weniger Sekunden erstarrt. Erfindungsgemäß kann davon ausgegangen werden, daß die Erstarrung innerhalb von 1 bis 3 Sekunden abgeschlossen ist. Dementsprechend bildet sich auf dem Verpackungsmaterial ein fester Film, die Schmelze erstarrt, bevor sie von den saugfähigen Verpackungsmaterialien aufgenommen werden kann. Dementsprechend führt die Beduftung nach dem erfindungsgemäßen Verfahren auch zu keinerlei Fleckenbildung. Da die Duftstoffzubereitung schnell erstarrt und nicht in das Verpackungsmaterial eindringt, kommt es auch nicht zu chemischen Reaktionen zwischen Duftstoffen und den Verpackungsmaterialien. Daher entstehen beim erfindungsgemäßen Verfahren auch die unangenehmen Duftnoten nicht, die sich ergaben, wenn nach bislang bekannten Verfahren beduftet wurde, wobei die Duftstoffe in die Verpackung eindrangen.

Weiter hat der Film in einer bevorzugten Ausführungsform der Erfindung den Effekt, die Durchstoßfestigkeit des Verpackungsmaterials zu erhöhen. Dies ist insbesondere im Hinblick auf die Stapelung der Verpackungen in Paletten und beim Transport auftretende Belastungen von Vorteil. In bevorzugten Ausführungsformen der Erfindung kann diese Erhöhung der Durchstoßfestigkeit schon wenige Minuten nach dem Auftragen der Zubereitung beobachtet werden.

Nach dem erfindungsgemäßen Verfahren wird zuerst die Dispersion des Polymeren mit dem Carbonsäuresalz hergestellt, wie beispielsweise in den Patentanmeldungen DE 18 11 466 und WO 91/00322 beschrieben. Die Dispersion ist bei Raumtemperatur fest, d.h. jedoch im Sinne der Erfindung nicht, daß die Dispersion zwingend einen definierten Schmelzpunkt haben muß, sondern lediglich, daß diese Dispersion bei Raumtemperatur nicht fließt.

Anschließend wird die Dispersion auf eine Temperatur zwischen 40 und 85 °C erhitzt und das Parfumöl bei dieser Temperatur in die Dispersion eingearbeitet. Die Einarbeitung erfolgt dabei bevorzugt durch langsame Zugabe des Parfumöls zur Dispersion unter Rühren bis der gewünschte Anteil Parfümöl in der Mischung enthalten ist. Es werden Parfumölgehalte von 0,1 bis 60 Gew.-%, bezogen auf die gesamte Mischung, angestrebt, wobei in bevorzugten Ausführungsformen der Erfindung hohe Parfumölgehalte von oberhalb 10, vorzugsweise oberhalb 20 Gew.-%, erwünscht sind, wobei Parfumölgehalte in dem Bereich von 30 - 50 Gew.-% besonders bevorzugt sind. Dabei kann das Parfumöl bei Raumtemperatur eingesetzt werden, es kann jedoch auch bevorzugt sein, das Parfumöl selbst auf eine Temperatur von 40 - 60°C vorzuheizen, um eine Viskositätserhöhung der Dispersion durch die Abkühlung bei der Zugabe zu verhindern. Insbesondere wenn der Anteil des einzuarbeitenden Parfumöls an der Dispersion groß ist, d. h. wenn mehr als 30 Gew.-% der resultierenden Mischung aus Parfumöl bestehen sollen, kann es daher bevorzugt sein, das Öl vorher zu temperieren. Nach der Zugabe des Parfumöls kann die Mischung zur Lagerung auf Raumtemperatur abgekühlt werden, oder noch heiß direkt weiter verarbeitet werden. Bei Raumtemperatur ist auch diese Mischung fest, d.h. im Zusammenhang dieser Erfindung, die Mischung fließt bei Raumtemperatur nicht.

Die Auftragung der Zubereitung auf die Verpackung erfolgt wiederum bei erhöhter Temperatur, um die gewünschte Viskosität zur Verarbeitung einstellen zu können. Bei der Verarbeitungstemperatur, die erfindungsgemäß bei mindestens 50 °C liegt, besitzt die Mischung eine Viskosität (gemessen mit einem schubspannungsgesteuerten Rotationsviskosimeter bei einer Scherrate von 100 s⁻¹) von maximal 10000 mPas. Nach oben ist die Verarbeitungstemperatur durch die Flüchtigkeit der Parfümöle begrenzt, es kann maximal bei 85°C gearbeitet werden. Die bevorzugte Arbeitstemperatur liegt bei 70 bis 80°C. Die Auftragung kann dabei mittels üblicher Technologien, beispielsweise durch Streichen oder Sprühen erfolgen. Erfindungsgemäß bevorzugt ist die Auftragung in einem Spinnsprühverfahren.

In einem solchen Spinnsprühverfahren wird die Zubereitung beim Austreten aus einer Düse durch gezielte Verwirbelung mit Druckluft in eine spiralförmige Bewegung versetzt, durch die eine flächendeckende und gleichzeitig kantenscharfe Auftragung ermöglicht wird. Die im Spinnsprühverfahren verwendete Technologie ist an sich bekannt und wird beispielsweise zum Auftragen von Schmefzkfebstoffen eingesetzt. Bevorzugt wird dabei nicht mehr als 1 g der Zubereitung auf die Verpackung aufgebracht.

Geeignete Verpackungsmaterialien zur Beduftung nach dem erfindungsgemäßen Verfahren sind dabei insbesondere solche aus Papier und/oder Pappe, wobei bevorzugt Faltschachteln beduftet werden. Insbesondere ist es dabei bevorzugt, solche Faltschachteln auf der Innenseite des Deckels zu beduften, da dann bei Öffnen der Verpackung der Dufteindruck besonders intensiv ist. Dabei erfolgt die Beduftung bevorzugt, nachdem die Schachtel gefaltet wurde, und ganz besonders bevorzugt, unmittelbar bevor die Verpackung verschlossen wird. Denkbar ist nach dem erfindungsgemäßen Verfahren jedoch auch die Beduftung von Kunststoffmaterialien, insbesondere Kunststoffolien.

Als Parfümöle bzw. Duftstoffe können im erfindungsgemäßen Verfahren einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, ∝-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosenoder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Die allgemeine Beschreibung der einsetzbaren Parfüme (siehe oben) stellte allgemein die unterschiedlichen Substanzklassen von Riechstoffen dar. Um wahrnehmbar zu sein, muß ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw.

Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Die oben beschriebene Ausführungsform der vorliegenden Erfindung, in der die leichter flüchtigen Riechstoffe bzw. Duftstoffe in eine Polymerzubereitung inkorporiert werden, ist eine solche Methode zur Riechstoffixierung. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.

Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanangaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschouliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-ÖI, Ysop-ÖI, Zimtöl, Zimtblätteröl, Zitronellol, Zitronenöl sowie Zypressenöl.

Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decyialdehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-nnonylketon, Muskon, β-Naphtholethylether, β-Naphtholmethylether, Nerol, Nitrobenzol, n-Nonylaldehyd, Nonylalkohol, n-Octylaldehyd, p-Oxy-Acetophenon, Pentadekanolid, β-Phenylethylalkohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester, Zimtsäurebenzylester.

Zu den leichter flüchtigen Riechstoffen zählen insbesondere die niedriger siedenden Riechstoffe natürlichen oder synthetischen Ursprungs, die allein oder in Mischungen eingesetzt werden können. Beispiele für leichter flüchtige Riechstoffe sind Alkylisothiocyanate (Alkylsenföle), Butandion, Limonen, Linalool, Linaylacetat und - propionat, Menthol, Menthon, Methyl-n-heptenon, Phellandren, Phenylacetaldehyd, Terpinylacetat, Zitral, Zitronellal.

Ein wesentlicher Bestandteil der Polymer-Zubereitung ist ein Carbonsäuresalz. Eine Aufgabe dieser Salze ist die Stabilisierung der Emulsion aus wäßriger Polymerzubereitung und Parfumöl. Um diese Wirkung zu erreichen, werden die Carbonsäuresalze bevorzugt in Mengen von 1 bis 20 Gew.-%, bezogen auf die gesamte Zubereitung, eingesetzt, wobei insbesondere ein Gehalt von 2 bis 10 Gew.-% zu besonders guten Ergebnissen führt. Als Carbonsäuresalz werden insbesondere Alkali- bzw. Ammoniumsalze aliphatischer Carbonsäuren mit 8 bis 36 Kohlenstoffatomen eingesetzt. Die Carbonsäuren können verzweigt oder unverzweigt sein und Doppelbindungen oder einfache Substituenten wie Chlor oder Brom enthalten. Bevorzugt werden aliphatische Monocarbonsäuren, insbesondere Fettsäuren mit 12 bis 22 Kohlenstoffatomen, eingesetzt. Dabei können sowohl Fettsäuren, die aus natürlichen Fetten oder Ölen gewonnen werden, als auch künstlich hergestellte Fettsäuren eingesetzt werden. Besonders günstige Ergebnisse wurden mit gesättigten Fettsäuren, beispielsweise Natriumstearaten erhalten. Bevorzugt sind im allgemeinen jedoch auch die Natriumsalze der anderen Fettsäuren mit 12 bis 22 Kohlenstoffatomen. Es können jedoch auch die Lithium-, Kalium- oder ggf. Alkyl-substituierten Ammoniumsalze eingesetzt werden.

Aufgrund der emulgierenden Wirkung der Carbonsäuresalze ist der Einsatz weiterer Emulgatoren, oder auch von Weichmachern, in den erfindungsgemäßen Mitteln nicht erforderlich. In speziellen Ausführungsformen kann es jedoch durchaus erwünscht sein, daß zusätzliche Emulgatoren und/oder Weichmacher in den Rezepturen enthalten sind. Geeignete Substanzen sind in den Patentanmeldungen DE 18 11 466 und WO 91/00322 beschrieben.

Als Polymerkomponente werden filmbildende, wasserlösliche bzw. wasserdispergierbare Polymere, die sowohl natürlichen als auch synthetischen Ursprungs sein können, eingesetzt. Dabei kann es bevorzugt sein, wenn Gemische verschiedener Polymere eingesetzt werden.

Geeignet sind beispielsweise Salze von Polyacryl- und Methacrylsäuren, Polyacrylamide bzw. Mischpolymerisate von Acrylamid mit N-alkylsubstituierten Acrylamiden sowie Polyvinylpyrrolidon, Besonders günstige Ergebnisse werden mit Polyvinylpyrrolidon eines Molekulargewichts von ca. 500.000 bis 900.000 g/mol erzielt. Weiterhin können Cellulosederivate wie Methyl- und Ethylcellulose, Oxyethylcellulose, Carboxymethylcellulose, abgebaute wasserlösliche Stärke und ethoxylierte und propoxylierte Stärkederivate, wie polymere, ethoxylierte Glucose, Carboxymethylstärke und dergl. mehr verwendet werden. Außerdem können als filmbildende Komponente Alginate oder Dextran zugesetzt werden.

Ferner können als wasserdispergierbare Polymere beispielsweise Phenolharze, Alkydharze und mit Leinöl, Ricinen, Ricinusöl, Sojaöl, Kokosöl, Tallöl und Fischöl modifizierte Alkylharze, acrylierte Alkydharze, Polyvinylacetale, Polyvinylacetate, Polyvinylbutyrat, Polyvinyläther, Polyvinylchlorid, Mischpolymerisate des Vinylchlorids mit Vinylidenchlorid sowie Polyacrylsäureester und Polymethacrylsäureester eingesetzt werden.

Die erfindungsgemäßen Duftstoffzubereitungen enthalten in einer bevorzugten Ausführungsform eine wäßrige Polyurethan-Dispersion als Polymerkomponente. Unter Polyurethan-Dispersionen werden hier ganz breit Reaktionsprodukte von mehrfunktionellen Alkoholen, Aminoalkoholen oder Aminen einerseits, mit mehrfunktionellen Isocyanaten andererseits verstanden, die entweder zusätzlich in die Kette einreagierte Bausteine enthalten, die nach Neutralisation ionische Gruppen bilden können und zusätzlich oder an Stelle solcher Gruppen hydrophile nichtionische Bestandteile enthalten, wodurch die Polymeren bei Wasserzugabe selbstdispergierend sind. Im Sinne der Erfindung sind besonders feinteilige Polyurethan-Dispersionen bevorzugte Ausgangsmaterialien, die opak durchscheinend bis durchsichtig aussehen, und bei denen das Polymere zumindest anteilsweise in Lösung vorliegen dürfte. Dabei gilt das Fachwissen des Polyurethan-Fachmannes, der über den Anteil an ionischen und/oder nicht-ionischen Bestandteilen die Feinteiligkeit beeinflussen kann.

Die als synthetische Polymere eingesetzten Polyurethan-Dispersionen enthalten als Ausgangsprodukt einreagiert ein Polyol oder eine Polyolmischung. Ganz allgemein gilt hier, daß diese Polyole über zumindest zwei reaktionsfähige Wasserstoffatome verfügen müssen und im wesentlichen linear sind. Dabei liegt das Molekulargewicht zwischen 300 und 40.000, vorzugsweise zwischen 500 und 6.000. Eingesetzt werden können Polyesterpolyole, Polyacetalpolyole, Polyetherpolyole, Polythioetherpolyole, Polyamidpolyole oder Polyesteramidpolyole mit jeweils 2 bis 4 Hydroxylgruppen, die auch teilweise durch Aminogruppen ausgetauscht sein können.

Darüber hinaus können als nichtionische hydrophile Modifizierungsmittel noch zusätzlich monofunktionelle Alkohole, insbesondere Etheralkohole eingesetzt werden. Bevorzugt sind dabei die Umsetzungsprodukte von C₁ - C₁₀-Alkoholen mit Ethylenoxid im Molekulargewichtsbereich bis 20 000, vorzugsweise 200 bis 6 000.

Als Polyether seien z.B. die Polymerisationsprodukte des Ethylenoxids, Propylenoxids, Butylenoxids sowie ihre Misch- oder Pfropfpolymerisationsprodukte sowie die durch Kondensation von mehrwertigen Alkoholen oder Mischungen derselben und die durch Alkoxylierung von mehrwertigen Alkoholen, Aminen, Polyaminen und Aminoalkoholen gewonnenen Polyether genannt. Auch isotaktisches Polypropylenglykol kann Verwendung finden.

Bevorzugtes Polyetherpolyol ist das Polytetrahydrofuran. Unter Polytetrahydrofuran werden hier Polyether verstanden, die theoretisch oder tatsächlich durch ringöffnende Polymerisation von Tetrahydrofuran dargestellt werden können, und an beiden Kettenenden jeweils eine Hydroxylgruppe aufweisen. Geeignete Produkte haben dabei einen Oligomerisationsgrad von ca. 1,5 bis 150, vorzugsweise von 5 bis 100.

Eine weitere bevorzugte Klasse von Polyolen sind Polycarbonatpolyole. Bevorzugt sind hier aliphatische Polycarbonatpolyole, also Ester der Kohlensäure mit difunktionellen Alkoholen der Kettenlänge C₂ bis C₁₀. Weniger geeignet sind Polycarbonatpolyole auf Basis Kohlensäure und Bisphenol-A.

Als Polyacetale kommen z. B. die aus Glykolen wie Diethylenglykol, Triethylenglykol, 4,4'-Dioxethoxy-diphenyl-dimethylmethan, Hexandiol und Formaldehyd herstellbaren Verbindungen in Frage. Auch durch Polymerisation cyclischer Acetale lassen sich geeignete Polyacetale herstellen.

Unter den Polythioethern seien insbesondere die Kondensationsprodukte von Thiodiglykol mit sich selbst und/oder mit anderen Glykolen, Dicarbonsäuren, Formaldehyd, Aminocarbonsäuren oder Aminoalkoholen angeführt. Je nach den Co-Komponenten handelt es sich bei den Produkten um Polythioether, Polythiomischether, Polythioetherester, Polythioetheresteramide. Derartige Polyhydroxylverbindungen können auch in alkylierter Form bzw. in Mischung mit Alkylierungsmitteln angewandt werden.

Zu den Polyestern, Polyesteramiden und Polyamiden zählen die aus mehrwertigen gesättigten und ungesättigten Carbonsäuren bzw. deren Anhydriden und mehrwertigen gesättigten und ungesättigten Alkoholen, Aminoalkoholen, Diaminen, Polyaminen und ihren Mischungen gewonnenen, überwiegend linearen Kondensate, sowie z.B. Polyterephthalate oder Polycarbonate. Auch Polyester aus Lactonen, z. B. Caprolacton oder aus Hydroxycarbonsäuren sind verwendbar. Die Polyester können Hydroxyl- oder Carboxylendgruppen aufweisen. Zu ihrem Aufbau können als Alkoholkomponente auch höhermolekulare Polymerisate oder Kondensate, wie z.B. Polyether, Polyacetale, Polyoxymethylene (mit)verwendet werden.

Auch bereits Urethan- oder Harnstoffgruppen enthaltende Polyhydroxylverbindungen sowie gegebenenfalls modifizierte natürliche Polyole wie Rizinusöl sind verwendbar. Grundsätzlich kommen auch Polyhydroxylverbindungen, welche basische Stickstoffatome aufweisen, in Frage, z. B. polyalkoxylierte primäre Amine oder Polyester bzw. Polythioether, welche Alkyl-diethanolamin einkondensiert enthalten. Weiterhin eingesetzt werden können Polyole, die durch vollständige oder teilweise Ringöffnung epoxidierter Triglyceride mit primären oder sekundären Hydroxylverbindungen erzeugt werden können, beispielsweise das Umsetzungsprodukt von epoxidiertem Sojaöl mit Methanol.

Als Polyisocyanate in den erfindungsgemäßen PU-Dispersionen sind alle aromatischen und aliphatischen Diisocyanate geeignet, wie z. B. 1,5-Naphthalendiisocyanat, 4.4'-Diphenylmethandiisocyanat, 4,4'-Diphenyldimethylmethandiisocyanat, Di- und Tetraalkyldiphenylmethandiisocyanat, 4,4'-Dibenzyldiisocyanat, 1,3-Phenylendiisocyanat, 1,4-Phenylendiisocyanat, die Isomeren des Toluylendiisocyanats, gegebenenfalls in Mischung, 1-Methyl-2,4-diisocyanato-cyclohexan, 1,6-Diisocyanato-2,2,4-trimethylhexan, 1,6-Diisocyanato-2,4,4-trimethylhexan, 1-lsocyanatomethyl-3-isocyanato-1,5,5-trimethyl-cyclohexan, chlorierte und bromierte Diisocyanate, phosphorhaltige Diisocyanate, 4,4'-Diisocyanatophenylperfluorethan, Tetramethoxy-butan-1,4-diisocyanat, Butan-1,4-diisocyanat, Hexan-1,6-diisocyanat, Dicyclohexylmethan-diisocyanat, Cyclohexan-1,4-diisocyanat, Ethylen-diisocyanat, Phthalsäure-bis-isocyanatoethylester, ferner Polyisocyanate mit reaktionsfähigen Halogenatomen, wie 1-Chlormethylphenyl-2,4-diisocyanat, 1-Brommethylphenyl-2,6-diisocyanat, 3,3-Bis-chlormethylether-4,4'diphenyldiisocyanat. Schwefelhaltige Polyisocyanate erhält man beispielsweise durch Umsetzung von 2 mol Hexamethylen-diisocyanat mit 1 mol Thiodiglykol oder Dihydroxydihexylsulfid. Weitere wichtige Diisocyanate sind Trimethylhexamethylendiisocyanat, 1,4-Diisocyanatobutan, 1,2-Diisocyanatododecan und Dimerfettsäure-diisocyanat. Die vorgenannten Isocyanate können allein oder auch in Mischung eingesetzt werden. Bevorzugt sind cyclische oder verzweigte aliphatische Diisocyanate wie Isophorondiisocyanat, aber auch Hexamethylendiisocyanat. Unter den aromatischen Diisocyanaten ist Tetramethylxyloldiisocyanat (TMXDI) bevorzugt.

Beim Aufbau der erfindungsgemäß eingesetzten Polyurethan-Dispersionen können auch Kettenverlängerungsmittel mit reaktionsfähigem Wasserstoff eingesetzt werden.

Zu den Kettenverlängerungsmitteln mit reaktionsfähigen Wasserstoffatomen zählen:
- die üblichen gesättigten und ungesättigten Glykole, wie Ethylenglykol oder Kondensate des Ethylenglykols, Butandiol-1,3, Butandiol-1,4, Butendiol, Propandiol-1,2, Propandiol-1,3, Neopentylglykol, Hexandiol, Bis-hydroxymethyl-cyclohexan, Dioxyethoxyhydrochinon, Terephthalsäure-bis-glykolester, Bernsteinsäure-di-2-hydroxyethyl-amid, Bernsteinsäure-di- N-methyl-(2-hydroxy-ethyl)-amid, 1,4-Di(2-hydroxy-methyl-mercapto)-2,3,5,6-tetrachlorbenzol, 2-Methylenpropandiol-(1,3), 2-Methyl-propandiol-(1,3);
- aliphatische, cycloaliphatische und aromatische Diamine wie Ethylendiamin, Hexamethylendiamin, 1,4-Cyclohexylendiamin, Benzidin, Diamino-diphenylmethan, Dichlor-diamino-diphenylmethan, die Isomeren des Phenylendiamins, Hydrazin, Ammoniak, Carbohydrazid, Adipinsäure-dihydrazid, Sebacinsäuredihydrazid, Piperazin, N-Methyl-propylendiamin, Diaminodiphenylsulfon, Diaminodiphenylether, Diaminodiphenyldimethylmethan, 2,4-Diamino-6-phenyltriazin;
- Aminoalkohole wie Ethanolamin, Propanolamin, Butanolamin, N-Methylethanolamin, N-Methyl-isopropanolamin;
- aliphatische, cycloaliphatische, aromatische und heterocyclische Mono- und Diaminocarbonsäuren wie Glycin, 1- und 2-Alanin, 6-Aminocapronsäure, 4-Aminobuttersäure, die isomeren Mono- und Diaminobenzoesäuren, die isomeren Mono- und Diaminonaphthoesäuren;
- Wasser

Es ist hervorzuheben, daß im Rahmen vorliegender Erfindung nicht streng zwischen den Verbindungen mit reaktionsfähigen Wasserstoffatomen mit einem Molekulargewicht von 300 bis 20 000 und den sogenannten "Kettenverlängerungsmitteln" unterschieden werden kann, da die Übergänge zwischen den beiden Verbindungsklassen fließend sind. Verbindungen, die nicht aus mehreren Monomereinheiten aufgebaut sind, jedoch ein Molekulargewicht über 300 aufweisen, wie z.B. 3,3'-Dibrom-4,4'-diaminodiphenylmethan, werden zu den Kettenverlängerungsmitteln gerechnet, ebenso jedoch Pentaethylenglykol, obwohl letzteres seiner Zusammensetzung nach eigentlich ein Polyetherdiol ist.

Spezielle Kettenverlängerungsmittel mit mindestens einem basischen Stickstoffatom sind z.B. mono-, bis- oder polyoxalkylierte aliphatische, cycloaliphatische, aromatische oder heterocyclische primäre Amine, wie N-Methyldiethanolamin, N-Ethyl-diethanolamin, N-Propyl-diethanolamin, N-lsopropyl-diethanolamin, N-Butyl-diethanolamin, N-lsobutyldiethanolamin, N-Oleyl-diethanolamin, N-Stearyl-diethanolamin, oxethyliertes Kokosfettamin, N-Allyl-diethanolamin, N-Methyl-diisopropanolamin, N-Ethyl-diisopropanolamin, N-Propyl-diisopropanolamin, N-Butyl-diisopropanolamin, N-Cyclohexyl-diisopropanolamin, N,N-Dioxethylanilin, N,N-Dioxethyltoluidin, N,N-Dioxethyl-1-aminopyridin, N,N'-Dioxethyl-piperazin, Dimethyl-bis-oxethylhydrazin, N,N'-Bis-(2-hydroxy-ethyl)-N,N'diethyl-hexahydro-p-phenylendiamin, N-12-Hydroxyethyl-piperazin, polyalkoxylierte Amine wie oxypropyliertes Methyl-diethanolamin, ferner Verbindungen wie N-Methyl-N,N-bis-3-aminopropylamin, N-(3-Aminopropyl)-N,N'-dimethylethylendiamin, N-(3-Aminopropyl)-N-methyl-ethanolamin, N,N'-Bis-(3-aminopropyl)-N, N'-dimethylethylendiamin, N,N'-Bis(3-aminopropyl)-piperazin, N-(2-Aminoethyl)-piperazin, N,N'-Bisoxyethylpropylendiamin, 2,6-Diaminopyridin, Diethanolamino-acetamid, Diethanolamidopropionamid, N,N-Bis-oxyethyl-phenyl-thiosemicarbazid, N,N-Bis-oxethyl-methyl-semicarbazid, p,p'-Bis-aminomethyl-dibenzylmethylamin, 2,6-Diaminopyridin, 2-Dimethylaminomethyl-2-methyl-propandiol-1,3.

Die den erfindungsgemäß vorzugsweise eingesetzten Polyurethan-Dispersionen zugrundeliegenden Polyurethane haben weiterhin als wichtigen Bestandteil eine in wäßriger Lösung zur Salzbildung befähigte funktionelle Komponente. Als solche können eingesetzt werden Dihydroxy- oder auch Diamino-Verbindungen, die eine ionisierbare Carbonsäure-, sulfonsäure- oder Ammoniumgruppe enthalten. Diese Verbindungen können entweder als solche eingesetzt werden oder sie können in-situ hergestellt werden. Um ionisierbare Carbonsäuregruppen tragende Verbindungen in das Polyurethan einzubringen, kann der Fachmann den Polyolen Dihydroxycarbonsäuren zugeben. Eine bevorzugte Dihydroxycarbonsäure ist beispielsweise die Dimethylolpropionsäure.

Um zur Salzbildung befähigte Sulfonsäuregruppen einzuführen, kann den Polyolen eine Diaminosulfonsäure zugesetzt werden. Beispiele sind 2,4-Diaminobenzolsulfonsäure aber auch die N-(w-Amino-alkan)-w'-aminoalkansulfonsäuren wie sie in der DE 20 35 732 beschrieben sind.

Erfindungsgemäß ist es bevorzugt, die zu verwendenden Polyurethan-Prepolymeren mit Hilfe von Carbonsäure- oder Sulfonsäuregruppen wasserlöslich zu machen, wobei gleichzeitig mit oder an Stelle der anionischen Modifizierungsmittel auch die bereits erwähnten nicht-ionischen Modifizierungsmittel, also zum Beispiel die Etheralkohole oder deren Derivate eingesetzt werden können.

Die zur Salzbildung befähigten Gruppen können durch Gegenionen teilweise oder vollständig neutralisiert werden. Auch ein Überschuß an Neutralisationsmittel ist möglich.

Dabei erfolgt die Herstellung der für die erfindungsgemäßen Zwecke insbesondere geeigneten Polyurethane wie in der Patentanmeldung WO 91/00322 beschrieben. Bei den in dieser Anmeldung angegebenen Zusammensetzungen für Polyurethandispersionen handelt es sich um im Sinne der vorliegenden Erfindung einsetzbare Polymerdispersionen. Auch die Herstellung der wäßrigen Dispersionen dieser Polymere, die bevorzugt klar bis opak sind, wird dort beschrieben. Der Fachmann muß dabei auf ein bestimmtes Verhältnis zwischen der zur Salzbildung befähigten Komponente und den übrigen, das Polyurethan aufbauenden Stoffen achten Anstelle der Modifizierung mit zur Salzbildung befähigten Komponenten kann auch eine nicht-ionische Modifizierung stattfinden. Zur nicht-ionischen Modifizierung eignen sich in erster Linie Monoalkohole, die durch Umsetzung von primären Alkoholen mit Ethylenoxid erhalten werden.

Bei der Herstellung der erfindungsgemäßen Polyurethan-Dispersionen, in die das Parfümöl eingearbeitet werden soll, hat der Fachmann auf das Verhältnis von Hydroxylgruppen zu Isocyanatgruppen zu achten. Dieses kann zwischen 1,0 : 0,8 und 1,0 : 4,0 liegen. Bevorzugt sind Verhältnisse von 1,0 : 1,1 bis 1,0 : 2,0, insbesondere 1,0 : 1,1 bis 1,0 : 1,8. Entsprechende Polyurethan-Dispersionen können in einem weiten Konzentrationsbereich hergestellt werden. Bevorzugt sind Zubereitungen mit einem Polymergehalt vor Einarbeitung des Parfumöls zwischen 20 und 80 Gew.-%, insbesondere 35 bis 60 Gew.-%.

### Beispiele:

Zur Untersuchung ihrer Eignung zur Beduftung von Verpackungen wurden verschiedene Polymer-Zubereitungen verwendet. Die Rezepturen A-G wurden zur Herstellung erfindungsgemäßer Zubereitungen eingesetzt, während die Rezepturen H bzw. I für Beispiele nicht erfindungsgemäßer Rezepturen stehen. Dabei besitzen die eingesetzten Polyurethan-Zubereitungen Zusammensetzungen entsprechend der Patentanmeldung WO 91/00322. Die Differenz der Summe der angegebenen Inhaltsstoffe zu 100 Gew.-% wird durch zusätzlich enthaltene, übliche Weichmacher, Emulgatoren und nicht-wäßrige Lösungsmittel verursacht.

Zur Herstellung der Duftzubereitungen wurden die Polymer-Zubereitungen mit ausgewählten Duftstoffgemischen (Tabelle 2) versetzt. Die Verarbeitungsbedingungen und die Zusammensetzung einiger resultierender Zubereitungen ist in Tabelle 3 angegeben. Alle Mischungen sind bei Raumtemperatur fest und Entmischen sich beim Lagern nicht.

**Tabelle 1:**

| Polymer-Zubereitungen | | | | | | | | | |
|---|---|---|---|---|---|---|---|---|---|
| | A | B | C | D | E | F | G | H | I |
| Polymer-zubereitung | PU | PU | PU | PU | PVP | PVP | PVP / PGE | PVAc / PVA | PVAc |
| Mengenanteile [Gew.-%] | | | | | | | | | |
| Polymer | 40 | 60 | 38 | 42 | 26 | 32 | 30 | 31 | 50 |
| Wasser | 47 | 23 | 53 | 45 | 51 | 51 | 47 | 69 | 35 |
| Na-Myristat | 1 | - | 6 | - | 8 | - | 8 | - | - |
| Na-Palmitat | 3 | 3 | - | 3 | - | - | - | - | - |
| Na-Stearat | 3 | 4 | - | 3 | - | 7 | - | - | - |
| PU: Polyurethan | | | | | | | | | |
| PVP: Polyvinylpyrrolidon | | | | | | | | | |
| PVA: Polyvinylalkohol (Verseifungszahl 20) | | | | | | | | | |
| PVAc: Polyvinylacetat | | | | | | | | | |
| PGE: Polyglucosidether | | | | | | | | | |

**Tabelle 2:**

| Zusammensetzung der Parfümöle [Gew.-%]: | |
|---|---|
| **Parfümöl 1** | |
| Bergamotteöl | 15,0 |
| Dihydromyrcenol | 20,0 |
| Citronenöl Messina | 7,5 |
| Mandarinenöl | 2,5 |
| Orangenöl süß | 5,0 |
| Allylamylglycolat | 2,0 |
| Cyclovertal | 0,5 |
| Lavandinöl grosso | 2,5 |
| Muskateller Salbeiöl | 1,0 |
| Lilial | 2,0 |
| β-Damascone | 0,1 |
| Geraniumöl Bourbon | 3,0 |
| Hedione | 5,0 |
| Cyclohexylsalicylat | 4,0 |
| Vertofix Coeur | 10,0 |
| Iso-E-Super | 5,0 |
| Ambroxan | 1,6 |
| Ethylenbrassylat | 10,0 |
| Evernyl | 1,0 |
| Dipropylenglycol (DPG) | 2,3 |

| **Parfümöl 2** | |
|---|---|
| Phenylethylalkohol | 52,0 |
| Dimethylbenzylcarbinylacetat | 2,5 |
| Iraldein gamma | 5,0 |
| Phenylessigsäure | 0,5 |
| Geranylacetat | 2,0 |
| Benzylacetat | 3,0, |
| Rosenoxid L 10% in DPG | 2,5 |
| Romilat | 2,0, |
| Irotyl | 0,5 |
| Cyclohexylsalicylat | 20,0 |
| Floramat | 10,0 |

**Tabelle 3:**

| Zusammensetzung der erfindungsgemäßen Zubereitungen und deren Herstellungsbedingungen | | | | |
|---|---|---|---|---|
| | E1 | E2 | E3 | E4 |
| **Polymer-Zubereitung** | G | G | G | A |
| Anteil der Polymer-Zubereitung [Gew.-%] | 85 | 80 | 75 | 70 |
| Temperatur der Polymer-Zubereitung beim Mischen [°C] | 70 | 70 | 70 | 75 |
| **Parfümöl** | 1 | 1 | 1 | 2 |
| Anteil des Parfümöls [Gew.-%] | 15 | 20 | 25 | 30 |
| Parfümöl-Temperatur beim Mischen [°C] | 20 | 20 | 20 | 20 |
| Scherviskosität bei 50 °C [mPas] | 3880 | 1150 | 1000 | 11200 |
| Scherviskosität bei 80 °C [mPas] | 1820 | 430 | 160 | 8900 |

**Tabelle 3a:**

| Zusammensetzung von Vergleichsbeipielen | | |
|---|---|---|
| | **V1 V2** | |
| **Polymer-Zubereitung** | H | H |
| Anteil der Polymer-Zubereitung [Gew.-%] | 70 | 80 |
| Temperatur der Polymer-Zubereitung beim Mischen [°C] | 70 | 70 |
| **Parfümöl** | 1 | 1 |
| Anteil des Parfümöls [Gew.-%] | 30 | 20 |
| Parfümöl-Temperatur beim Mischen [°C] | 20 | 20 |

Die in Tabelle 3 angegebenen Viskositäten wurden mit einem schubspannungsgesteuerten Rotationsviskosimeter (Rheometer TA 1000 AR; Firma TA Instruments) bei den angegebenen Temperaturen und einer Scherrate von 100 s⁻¹ bestimmt.

Diese Zubereitungen wurden im Spinnsprühverfahren (Nordson CF-200 Auftragungskopf; Nordson 3400 V Kolbenpumpe) auf Testkartons aufgebracht. Dabei wurden jeweils etwa 0,5g der Zubereitung auf einer Länge von 190 mm verteilt. Anschließend wurden die Kartons optisch auf Fleckenbildung beurteilt, die Durchstoßfestigkeit gemessen, sowie der Dufteindruck der Verpackung (des Testkartons) beurteilt.

Keiner der mit den erfindungsgemäßen Mitteln bedufteten Kartons wies Flecken auf.

Zur Bestimmung der Durchstoßfestigkeit wird die Kraft gemessen, die aufgewendet wird, wenn ein Durchstoßkörper die eingespannte Probe durchdringt. Hierzu wurde das Probestück zwischen zwei Spannplatten befestigt und von einem Durchstoßkörper, der die Form einer rechtwinkeligen gleichseitigen Dreieckpyramide besitzt und an einem Pendel, das in Form eines Kreisbogens ausgebildet ist montiert ist, durchschlagen. Die Prüfung erfolgte bei 23°C und 50 % relativer Luftfeuchte. Um reproduzierbare Werte zu erhalten, wurden jeweils 10 Proben gegen die Oberseite und 10 Proben gegen die Unterseite geprüft. Die als Ergebnis angegebenen Werte sind Mittelwerte aus diesen jeweils 20 Einzelversuchen, wobei als Meßwert die Kraft angegeben ist, mit der der Karton gerade noch nicht durchstoßen wird. Für das Rohmaterial, dem unbehandelten Karton, ergab sich eine Durchstoßfestigkeit von 3,2 J. Im Vergleich wurde die Durchstoßfestigkeit von Kartons, die mit Zubereitungen behandelt waren, die 30 Gew.-% Parfumöl enthielten, untersucht (Tabelle 4). Die Durchstoßfestigkeit des Kartons, der mit einer nicht erfindungsgemäßen Zubereitung (V1) behandelt wurde, blieb nach dem Auftragen nahezu unverändert, während der mit der erfindungsgemäßen Polyurethan-Carbonsäuresalz-Parfum-Zubereitung (E4) behandelte Karton schon 5 Minuten nach dem Auftragen eine deutlich erhöhte Durchstoßfestigkeit aufwies.

**Tabelle 4:**

| Durchstoßfestigkeit der bedufteten Kartons (in J) | | |
|---|---|---|
| Durchstoßfestigkeit [J] | **E4** | **V1** |
| 5 min nach dem Auftragen | 3,9 | 3,2 |
| 15 min nach dem Auftragen | 3,9 | 3,3 |

Der subjektive Geruchseindruck des bedufteten Kartons wurde, anhand von Kartons die mit E4 beduftet waren, von Parfümeuren beurteilt. In Tabelle 5 ist die aufgetragene Menge der Duftstoffzubereitung angegeben, sowie der ermittelte Dufteindruck, der nach dem Auftragen bzw. nach der jeweils angegebenen Lagerzeit, ermittelt wurde. Auch nach 4 Wochen war der Duft noch sehr intensiv vorhanden. Der Duft läßt direkt nach dem Auftragen etwas nach, während der anschließenden Lagerung vermindert sich der noch intensiv vorhandene Dufteindruck jedoch nicht mehr. Der Beurteilung in Tabelle 5 liegt folgende Skala zugrunde:
- 1: kein Duft
- 2: schwacher Duft
- 3: mittel starker Duft
- 4: starker Duft
- 5: sehr starker Duft

**Tabelle 5:**

| Dufteindruck des mit E4 bedufteten Kartons | | |
|---|---|---|
| Aufgetragene Menge | 0,4 g | 0,5 g |
| Lagerzeit: | | |
| - sofort nach dem Auftragen | 4-5 | 5 |
| - 2 Wochen | 4 | 4-5 |
| - 4 Wochen | 4 | 4-5 |

## Patentansprüche

1. Verfahren zur Beduftung von Verpackungen wobei eine bei Raumtemperatur feste Zubereitung aus Parfüm- und/oder Aromaölen und Polymeren bei erhöhter Temperatur in geschmolzenem Zustand auf die Verpackung aufgebracht wird, **dadurch gekennzeichnet, daß** Parfüm- und/oder Aromaöle bei erhöhter Temperatur mit einer bei Raumtemperatur festen Dispersion, enthaltend mindestens ein Polymer und mindestens ein Carbonsäuresalz, in Wasser gemischt werden und diese bei Raumtemperatur ebenfalls feste Mischung bei einer Temperatur von mindestens 50 °C und maximal 85 °C auf die Verpackung aufgebracht wird, wobei die Mischung bei der Verarbeitungstemperatur und einer Scherrate von 100 s⁻¹ eine Viskosität von maximal 10000 mPas besitzt.

2. Verfahren nach Anspruch 1, **dadurch gekennzeichnet, daß** die Aufbringung auf die Verpackung im Spinnsprühverfahren erfolgt.

3. Verfahren nach einem der Ansprüche 1 oder 2, **dadurch gekennzeichnet, daß** als Carbonsäuresalz ein Salz einer Fettsäure, bevorzugt einer gesättigten Fettsäure eingesetzt wird.

4. Verfahren nach einem der Ansprüche 1 bis 3, **dadurch gekennzeichnet, daß** als Polymer ein Polyurethan-Polymer, insbesondere in Form einer wäßrigen Dispersion, eingesetzt wird.

5. Verfahren nach einem der Ansprüche 1 bis 3, **dadurch gekennzeichnet, daß** als Polymer ein Polyvinylpyrrolidon-Polymer eingesetzt wird.

6. Zubereitung zur Beduftung von Verpackungen, die Parfüm- und/oder Aromaöle sowie Polymere enthält und die bei Raumtemperatur fest ist und bei erhöhter Temperatur schmilzt, **dadurch gekennzeichnet, daß** sie bei mindestens 50°C und maximal 85°C und einer Scherrate von 100 s⁻¹ eine Viskosität von maximal 10000 mPas besitzt und 0,1 - 60 Gew.-% Parfüm- und/oder Aromaöl sowie 1 - 20 Gew.-% Carbonsäuresalz neben Polymer, Wasser und gegebenenfalls weiteren Hilfsstoffen enthält.

7. Zubereitung zur Beduftung von Verpackungen gemäß Anspruch 6, **dadurch gekennzeichnet, daß** sie als Carbonsäuresalz das Salz einer Fettsäure, bevorzugt einer gesättigten Fettsäure enthält.

8. Zubereitung zur Beduftung von Verpackungen gemäß einem der Ansprüche 6 oder 7, **dadurch gekennzeichnet, daß** sie ein Polyurethan-Polymer enthält.

9. Zubereitung zur Beduftung von Verpackungen gemäß einem der Ansprüche 6 oder 7, **dadurch gekennzeichnet, daß** sie ein Polyvinylpyrrolidon-Polymer enthält.

10. Zubereitung zur Beduftung von Verpackungen gemäß einem der Ansprüche 6 bis 9, **dadurch gekennzeichnet, daß** sie 30 - 50 Gew.-% Parfum- und/oder Aromaöl sowie 2 - 10 Gew.-% Carbonsäuresalz enthält.

## Claims

1. A process for perfuming packaging in which a preparation of perfume and/or aroma oils and polymers which is solid at room temperature is applied to the packaging in molten form at elevated temperature, **characterized in that** perfume and/or aroma oils are mixed at elevated temperature with a dispersion solid at room temperature containing at least one polymer and at least one carboxylic acid salt in water and the resulting mixture, which is also solid at room temperature, is applied to the packaging at a temperature of at least 50°C and at most 85°C, the mixture having a viscosity of at most 10,000 mPas at the processing temperature and at a shear rate of 100 s⁻¹.

2. A process as claimed in claim 1, **characterized in that** the mixture is applied to the packaging by spin spraying.

3. A process as claimed in claim 1 or 2, **characterized in that** a salt of a fatty acid, preferably a saturated fatty acid, is used as the carboxylic acid salt.

4. A process as claimed in any of claims 1 to 3, **characterized in that** a polyurethane polymer, more particularly in the form of an aqueous dispersion, is used as the polymer.

5. A process as claimed in any of claims 1 to 3, **characterized in that** a polyvinyl pyrrolidone polymer is used as the polymer.

6. A preparation for perfuming packaging which contains perfume and/or aroma oils and polymers and which is solid at room temperature and melts at elevated temperature, **characterized in that** it has a viscosity of at most 10,000 mPas at a temperature of at least 50°C and at most 85°C and at a shear rate of 100 s⁻¹ and contains 0.1 to 60% by weight perfume and/or aroma oil and 1 to 20% by weight carboxylic acid salt besides polymer, water and optionally other additives.

7. A preparation for perfuming packaging as claimed in claim 6, **characterized in that** it contains the salt of a fatty acid, preferably a saturated fatty acid, as the carboxylic acid salt.

8. A preparation for perfuming packaging as claimed in claim 6 or 7, **characterized in that** it contains a polyurethane prepolymer.

9. A preparation for perfuming packaging as claimed in claim 6 or 7, **characterized in that** it contains a polyvinyl pyrrolidone polymer.

10. A preparation for perfuming packaging as claimed in any of claims 6 to 9, **characterized in that** it contains 30 to 50% by weight perfume and/or aroma oil and 2 to 10% by weight carboxylic acid salt.

## Revendications

1. Procédé permettant de parfumer des emballages, dans lequel une préparation d'huiles parfumées et/ou aromatiques et de polymères, solide à température ambiante, est appliquée sur l'emballage à température augmentée à l'état fondu, **caractérisé en ce que** les huiles parfumées et/ou aromatiques sont mélangées dans l'eau à température augmentée avec une dispersion solide à température ambiante, contenant au moins un polymère et au moins un sel d'acide carboxylique, et **en ce que** ce mélange, également solide à température ambiante, est appliqué sur l'emballage à une température de 50 °C au minimum et de 85 °C au maximum, le mélange présentant une viscosité de 10 000 mPa.s au maximum à la température de mise en oeuvre et à un taux de cisaillement de 100 s⁻.

2. Procédé selon la revendication 1, **caractérisé en ce que** l'application sur l'emballage est effectuée par le procédé de pulvérisation en spire.

3. Procédé selon une des revendications 1 ou 2, **caractérisé en ce que** l'on utilise comme sel d'acide carboxylique, un sel d'un acide gras, de préférence d'un acide gras saturé.

4. Procédé selon une des revendications 1 à 3, **caractérisé en ce que** l'on utilise comme polymère, un polymère de polyuréthane, en particulier sous la forme d'une dispersion aqueuse.

5. Procédé selon une des revendications 1 à 3, **caractérisé en ce que** l'on utilise comme polymère, un polymère de polyvinylpyrrolidone.

6. Préparation permettant de parfumer des emballages, qui contient des huiles parfumées et/ou aromatiques et des polymères et qui est solide à température ambiante et fond à température augmentée, **caractérisée en ce qu'**elle présente une viscosité de 10 000 mPa.s au maximum à 50 °C au minimum et à 85 °C au maximum et à un taux de cisaillement de 100 s⁻¹ et **en ce qu'**elle contient 0,1 à 60 % en poids d'huile parfumée et/ou aromatique ainsi que 1 à 20 % en poids de sel d'acide carboxylique, de même qu'un polymère, de l'eau et éventuellement d'autres adjuvants.

7. Préparation permettant de parfumer des emballages selon la revendication 6, **caractérisée en ce qu'**elle contient comme sel d'acide carboxylique le sel d'un acide gras, de préférence un acide gras saturé.

8. Préparation permettant de parfumer des emballages selon une des revendications 6 ou 7, **caractérisée en ce qu'**elle contient un polymère de polyuréthane.

9. Préparation permettant de parfumer des emballages selon une des revendications 6 ou 7, **caractérisée en ce qu'**elle contient un polymère de polyvinylpyrrolidone.

10. Préparation permettant de parfumer des emballages selon une des revendications 6 à 9, **caractérisée en ce qu'**elle contient 30 à 50 % en poids d'huile parfumée et/ou aromatique et 2 à 10 % en poids de sel d'acide carboxylique.
